# NEUE EUROPÄISCHE PATENTSCHRIFT

(11) **EP 0 438 512 B2**
(45) Veröffentlichungstag und Bekanntmachung des Hinweises auf die Entscheidung über den Einspruch: **27.07.2005**
(45) Hinweis auf die Patenterteilung: 29.12.1997
(21) Anmeldenummer: 89912096.8
(22) Anmeldetag: 11.10.1989
(51) Int. Cl.: C12Q 1/68

(54) **VERFAHREN ZUR ANALYSE VON LÄNGENPOLYMORPHISMEN IN DNA-BEREICHEN**
PROCESS FOR ANALYSING LENGTH POLYMORPHISMS IN DNA DOMAINS
PROCEDE POUR L'ANALYSE DE POLYMORPHISMES LONGITUDINAUX DANS DES REGIONS D'ADN

(30) Priorität: 11.10.1988 DE 3834636
(43) Veröffentlichungstag der Anmeldung: 31.07.1991
(73) Patentinhaber: Max-Planck-Gesellschaft zur Förderung der Wissenschaften e.V., Berlin (DE)
(72) Erfinder: JÄCKLE, Herbert, D-7400 Tübingen (DE); TAUTZ, Diethard, D-8000 München 60 (DE)
(74) Vertreter: VOSSIUS & PARTNER
(86) Internationale Anmeldenummer: PCT/EP1989/001203
(87) Internationale Veröffentlichungsnummer: WO 1990/004040

(56) Entgegenhaltungen:
- EP-A- 0 237 362
- SCIENCE, Band. 240, 1988 Jean L. Marx: "Multiplying Genes by Leaps and Bounds
- Chemical Abstracts, Band 108, no. 1, 4 Januar 1988, (Columbus, Ohio, US), Rollo, F et al : "Polymerase chain reaction fingerprints ", siehe Seite 154, Zusammenfassung 1552s, & Nucleic Acids Res. 1987, 15(21), 9094
- Nature 318 (1985) 577-579
- Nature 332 (1988) 278-281
- Nature 332 (1988) 543-546
- WO 89/07658
- Nucleic Acids Res 16 (1988) 5196
- Nature 322 (1986) 652-656
- Human Genet 74 (1986) 239-243
- Human Genet 79 (1988) 265-272
- Science 235 (1987) 1616
- Methods Enzymol 155 (1987) 335
- Am J Human Genet 44 (1989) 388-396
- J Forensic Sci 33 (1988) 1111-26
- PNAS 85 (1988) 6622-26
- Genomics 3 (1988) 249-256
- Am J Hum Genet 43 (1988) 249-256
- Nature 295 (1982) 31-35
- Nucleic Acids Res 15 (1987) 529
- Nucleic Acids Res 16 (1988) 10953-67

## Beschreibung

Die Erfindung betrifft ein Verfahren zur identitäts- und Verwandtschaftsbestimmung von Organismen auf der Basis von Längenpolymorphismen in Bereichen simpler oder kryptisch simpler DNA-Sequenzen.

Alle üblichen Verfahren zur Identitäts- und Verwandtschaftsbestimmung auf der Basis von DNA-Längenpolymorphismen beruhen auf der Verwendung von Restriktionsendonucleasen. Dabei werden spezifische DNA-Fragmente hergestellt, die anschließend mit Hybridisierungsverfahren nachgewiesen werden. Mit diesen Verfahren werden entweder Längenvariationen nachgewiesen, die zwischen den entsprechenden Erkennungsstellen für Restriktionsendon ucleasen entstanden sind, oder Längenvariationen, die aufgrund des Fehlens bestimmter Restriktionsspaltstellen entstanden sind. Das erstgenannte Verfahren macht sich die Längenvariation in sogenannten Minisatellitenbereichen (3, 4, 4a, 4b) bzw. in Bereichen mit spezifischen simplen DNA-Sequenzen (5) zunutze. Das zweite Verfahren, bei dem Restriktionslängenpolymorphismen (RFLP) nachgewiesen werden, ist nur in speziellen, empirisch gefundenen Fällen anwendbar und ist im wesentlichen nur sinnvoll einsetzbar bei der Analyse von genetischen Krankheiten.

EP-A 266 787 beschreibt ein Fingerprint-Verfahren zur Analyse von Längenpolymorphismen in DNA Bereichen, bei dem zahireiche Banden durch Hybridisierung mit Oligonucleotiden identifiziert werden. Die oligonucleotide enthalten Wiederholungseinheiten von 2 bis 4 Nucleotiden. Das in der EP-A2 266 787 beschriebene Fingerprint-Verfahren weist jedoch verschiedene Nachteile auf: hochmolekulare DNA muß isoliert werden, Gesamt-DNA-Blots müssen angefertigt werden, komplexe Bandenmuster müssen verglichen werden (Fingerprint), individuelle Allele können nicht bestimmt werden (man weiß nicht, welche Bande zu welchem Locus gehört) und die Größe der zu untersuchenden Fragmente (Banden) gestattet keine genaue Längenbestimmung (niederprozentige Agarosegele werden verwendet), so daß jegliche Vergleiche auf dem gleichen Gel durchgeführt werden müssen (keine Computerdatenbank anwendbar). Die EP-A2 266 787 beschreibt nicht, ob die Gegenstücke der repetitiv-DNA-Sonden in der genomischen DNA als solche längenpoymorph sind. Das beschriebene Verfahren beruht auf dem Vorhandensein weiterer oder der Abwesenheit vorher beobachteter Restriktionsspaltstellen, die keinesfalls notwendigerweise innerhalb repetitiver genomischer DNA-Sequenzen liegen müssen.

Der Nachteil der beiden bekannten Verfahren besteht darin, daß eine Hybridisierungsreaktion durchgeführt werden muß, um die längenpolymorphen Bereiche sichtbar zu machen. Dadurch werden die Verfahren zeitaufwendig und teuer. Weiterhin erlaubt eine einzelne Analyse mit den bisherigen Verfahren in der Regel keine ausreichend abgesicherte Aussage, so daß zusätzlich noch eine zweite, unabhängige Analyse erforderlich wird. Deshalb eignen sich diese Verfahren nicht sehr gut für Reihenuntersuchungen und Routinetests. Außerdem sind die beschriebenen Verfahren nicht zur Automatisierung geeignet.

Higuchi et al. (5a) beschreiben ein weiteres Verfahren zur Analyse eines längenpolymorphen Locus, das eine Primer-gesteuerte Polymerisationsreaktion bestimmter mitochondrialer DNA-Sequenzen umfaßt. Dieses Verfahren kann aufgrund der dabei verwendeten mitochondrialen Marker nicht zur Verwandtschaftsbestimmung verwendet werden.

Somit liegt der Erfindung die Aufgabe zugrunde, ein Verfahren zur Analyse von Längenpolymorphismen in DNA-Bereichen bereitzustellen, das hochempfindlich ist, bei geringem Zeitaufwand zuverlässige Ergebnisse liefert, auch für Reihenuntersuchungen und Routinetests geeignet ist, und gegebenenfalls auch automatisch durchgeführt werden kann.

Erfindungsgemäß wird diese. Aufgabe durch die Bereitsellung des Verfahrungsgemäß Anspruch 1 gelöst

Bei diesem Verfahren werden die einzelnen Primer-Moleküle des Primer-Paares in einem Abstand von 50 bis 500 Nucleotiden voneinander an den zu untersuchenden DNA-Bereich angelagert, so daß sie diesen im angegebenen Abstand umspannen. Dabei wird der zu untersuchende DNA-Bereich von den angelagerten Molekülen des Primer-Paares umgeben.

Die Primer-gesteuerte Kettenreaktion ist als solche aus der EP-A2 0 200 362 (1), aus der EP-A1 0 237 362 (la) und aus (2) bekannt. Dabei handelt es sich um ein Verfahren zur Amplifikation spezifischer DNA-Fragmente, bei dem eine PCR (Polymerase Chain Reaction) durchgeführt wird. Die spezifische Amplifikation wird bei diesem Verfahren durch die Verwendung von Oligonucleotid-Primern erzielt, die das Zielmolekül antiparallel flankieren. Dadurch werden bei einer matrizenabhängigen Verlängerung der Primer durch eine Polymerase DNA-Fragmente gebildet, die selbst wieder als Matrizen für einen erneuten Zyklus zur Verfügung stehen. Die DNA-Synthese wird durch Hitzedenaturierung des Ausgangsmoleküls, Anlagerung der entsprechenden Primer und durch Kettenverlängerung mit einer Polymerase eingeleitet. Durch eine erneute Hitzedenaturierung wird der nächste Zyklus begonnen. Der spezifisch amplifizierte Bereich wächst dadurch in exponentieller Weise und es wird schließlich ein durch normale Gel-elektrophorese nachweisbares Fragment gebildet. Die Länge dieses Fragments wird durch die 5'-Enden der Primer und dem dazwischenliegenden Bereich bestimmt. Die Verwendung von thermostabilen Synthesekomponenten erlaubt es, das Verfahren durch einfache und leicht automatisierbare Erhitzungs- und Kühlungszyklen zu steuern. Unter der "antiparallelen Flankierung" des Zielmoleküls durch Oligonucleotid-Primer versteht man die Anlagerung je eines der beiden Primer eines Primer-Paares an einen der komplementären Stränge des Zielmoleküls, so daß die 3'-Enden des Primer-Paares zueinander zeigen.

Marxs beschreibt in (15) verschiedene Anwendungen des PCR-Verfahrens.
Rollo et al. beschreiben in die Verwendung des PCR-Verfahrens zur Unterscheidung verschiedener Arten des pflanzenpatogenen Pilzes Phoma.
Die Verwendung von simplen und kryptisch simplen DNA-Sequenzen im Rahmen von PCR-Verfahren zur Identitätsund Verwandtschaftsbestimmung von Organismen wird in keiner dieser Druckschriften beschrieben.

Simple und kryptisch simple DNA-Sequenzen sind repetitive Bestandteile von allen eukaryontischen Genomen, die teilweise auch in prokaryontischen Genomen (6-9) vorkommen. Dabei umfassen simple DNA-Sequenzen kurze DNA-Motive, die mindestens drei Nucleotide und höchstens 6 Nucleotide enthalten und dutzend- bis hundertmal tandemartig wiederholt sind. Diese simplen DNA-Sequenzen sind durch Hybridisierung mit synthetischen DNA-Sequenzen und durch direkte Sequenzierung in allen bisher analysierten eukaryontischen Genomen und auch im menschlichen Genom gefunden worden (8, 10). Vermutlich kommen darin alle möglichen Permutationen von kurzen Motiven mit unterschiedlicher Häufigkeit vor (9). Kryptisch simple DNA-Sequenzen zeichnen sich durch eine überzufällig häufige, aber unregelmäßige direkte Wiederholung von kurzen DNA-Motiven aus (9). Kryptisch simple DNA-Sequenzen werden in der Regel nur indirekt mit einem entsprechenden Computerprogramm in bereits sequenzierten DNA-Bereichen gefunden. Sie treten jedoch mindestens ebenso häufig oder sogar noch häufiger auf, wie simple DNA-Sequenzen:
Die simplen und kryptisch simplen DNA-Sequenzen dürften durch genomische Mechanismen entstanden sein, die eine Tendenz haben, bereits existierende kurze Verdopplungen von beliebigen DNA-Sequenzmotiven nochmals zu verdoppeln oder in beliebigen DNA-Sequenzmotiven längere Bereiche von bereits bestehenden simplen oder kryptisch simplen DNA-Sequenzen teilweise zu deletieren (8-10). Daher ist davon auszugehen, daß diese Bereiche in der Regel längenpolymorph sind. Auf diesem Längenpolymorphismus beruht das erfindungsgemäße Verfahren.

Für das erfindungsgemäße Verfahren geeignete simple oder kryptisch simple DNA-Sequenzen können mit oder ohne Hilfe eines Computerprogramms in bereits bekannten DNA-Sequenzen gefunden werden (9). Geeignet ist eine simple oder kryptisch simple DNA-Sequenz dann, wenn sie eine Länge von ca. 20 bis 300 Nucleotiden besitzt, und von Zufallssequenzen, also von DNA-Sequenzen ohne interne Wiederholungen flankiert wird. Aus dem Bereich der flankierenden DNA-Sequenzen ohne interne Sequenzwiederholungen werden dann Stücke ausgewählt, zu denen geeignete komplementäre, synthetische Oligonucleotide hergestellt werden. Geeignet ist ein Oligonucleotid für diesen Zweck dann, wenn seine Nucleotidzusammensetzung und seine Nucleotidabfolge mit hoher Wahrscheinlichkeit nur einmal im zu untersuchenden Genom vorkommt und somit spezifisch für den individuell zu analysierenden DNA-Bereich ist.

Vorzugsweise werden im erfindungsgemäßen Verfahren Längenpolymorphismen von simplen oder kryptisch simplen DNA-Sequenzen untersucht, die im wesentlichen aus Trinucleotid-Motiven aufgebaut sind.
Bei der Untersuchung von Längenpolymorphismen solcher simplen oder kryptisch simplen DNA-Sequenzen werden sogenannte "slippage"-Artefakte vermieden. Diese treten beispielsweise bei von aus Dinucleotid-Motiven aufgebauten simplen oder kryptisch simplen DNA-Sequenzen auf. Dabei entstehen Reaktionsprodukte, die kürzer sind, als das erwünschte Hauptprodukt (vgl. Vergleichsbeispiel). Diese artifiziellen Banden können unter Umständen nur schwer von "echten" Banden unterschieden werden, was die Interpretation der Ergebnisse erschwert. Bei der Verwendung von simplen oder kryptisch simplen Trinucleotidsequenzen treten diese Artefakte nicht, oder in erheblich verringertem Maße auf (vgl. Beispiel 3).

In einer besonders bevorzugten Ausführungsform des erfindungsgemäßen Verfahrens ist die simple oder kryptisch simple DNA-Sequenz im wesentlichen aus dem Trinucleotid-Motiv ^{5'}CAG^{3'}/^{5'}CTG^{3'} aufgebaut.

Vorzugsweise werden bei dem erfindungsgemäßen Verfahren zwei Primer-Paare eingesetzt. In einer besonders bevorzugten Ausführungsform werden 2 bis 50 Primer-Paare eingesetzt.

Vorzugsweise haben die beim erfindungsgemäßen Verfahren verwendeten Primer eine Länge von 15 bis 25 Nucleotiden.

In einer bevorzugten Ausführungsform des erfindungsgemäßen Verfahrens werden bei der Verwendung mehrerer Primer-Paare die einzeinen Primer-Paare so ausgewählt, daß die entspraehenden spezifischen Polymerase-Kettenreaktionsprodukte der einzelnen Primer-Paare auf einem geeigneten Gel in einzelne Banden auftrennbar sind.

In einer anderen bevorzugten Ausführungsform des erfindungsgemäßen Verfahrens erfolgt der Nachweis der spezifischen Polymerasekettenreaktionsprodukte durch radioaktive Markierung oder durch nicht-radioaktive Markierung, z.B. mit Fluoreszenztarbstoffen.

Die Markierung der Oligonucleotid-Paare kann radioaktiv oder wie in (12) beschrieben, mit einem fluoreszierenden Farbstoff durchgeführt werden.

Ferner sind Bestecke (Kits), mit denen sich das erfindungsgemäße Verfahren durchführen läßt, Gegenstand der vorliegenden Erfindung. Die darin enthaltenen Primer sind gegebenenfalls radioaktiv, z.B mit ³⁵S oder ¹⁴C, oder fluoreszenz-markiert.

Die beim erfindungsgemäßen Verfahren erhaltenen Syntheseprodukte lassen sich mit hochauflösenden Gelsystamen, wie üblichen Sequenzierungsgelen, auftrennen. Dabei kann auch die Länge der Syntheseprodukte bestimmt werden. Polymorphismen, die durch Insertionen oder Deletionen einzelner oder mehrerer Motive der simplen oder kryptisch simplen DNA-Sequenz gebildet werden, sind durch eine geänderte Position der Syntheseprodukte im Gel erkennbar. Bei geeigneter Auswahl der Primer-Paare lassen sich bei einem geeigneten Auflösungsvermögen des Gelsystems ca. 20 bis 50 unabhängige polymorphe Bereiche gleichzeitig untersuchen. Somit läßt sich die Identität eines Individuums aufgrund der individuellen Kombination von Längenvertellungen der erhaltenen Syntheseprodukte zuverlässig feststellen.

Falls keine geeigneten simplen oder kryptisch simplen DNA-Sequenzen in den zu untersuchenden DNA-Bereichen bekannt sind, lassen sich diese wie folgt identifizieren.
Eine zu untersuchende genomische DNA wird einer partiellen Restriktionsspaltung unterworfen. Dabei werden Restriktionsenzyme verwendet, die üblicherweise nicht in simplen oder kryptisch simplen DNA-Sequenzen spalten. Die erhaltenen DNA-Fragmente werden in einem geeigneten Vektor, z.B. in lambda-Phagen-Derivaten oder in M13-Phagen cloniert und sodann in üblicher Weise durch Hybridisierung auf simple oder kryptisch simple DNA-Sequenzen abgesucht; vgl. (11). Die verwendeten Sondenmoleküle sind synthetische DNA-Moleküle, die verschiedene Permutationen von simplen oder kryptisch simplen DNA-Sequenzen enthalten. Auf diese Weise lassen sich hybridisierende Plaques identifizieren. Sodann kann die darin enthaltene rekombinante DNA isoliert und durch Sequenzierung charakterisiert werden. Die so erhaltene DNA-Sequenz läßt sich dann auf für das erfindungsgemäße Testverfahren geeignete DNA-Sequenzen absuchen.

Das erfindungsgemäße Verfahren wurde mit Drosophila-DNA als Modellsystem ausgeführt. Da jedoch simple und kryptische simple DNA-Sequenzen in allen eukaryontischen Genomen und teilweise auch in prokaryontischen Genomen vorkommen, ist davon auszugehen, daß die im Drosophila-Modellsystem erzielten Ergebnisse auch bei der Untersuchung anderer Genome, insbesondere bei der Untersuchung des menschlichen Genoms erzielt werden können.

Somit ist das erfindungsgemäße Verfahren für die Identitäts- und Verwandtschaftsbestimmung von Organismen, beispielsweise von Menschen geeignet.
Beim Menschen lassen sich Vaterschaftstests und forensische Tests zur Identitätsbestimmung von Straftätem mit dem erfindungsgemäßen Verfahren durchführen; vgl. auch Vergleichsbeispiel 1

Neben der Identitätsbestimmung für Individuen ist das Verfahren auch geeignet, den Vererbungsgang für genetische Krankheiten zu bestimmen, für die der Locus bekannt und sequenziert ist. Dazu werden eine oder mehrere simple oder kryptisch simple Sequenzen ausgewählt, die im oder in der Nähe des zu analysierenden Locus liegen. Das spezifische Längenmuster dieser Bereiche wird mit dem mutierten Locus korreliert, so wie dies mit herkömmlichen RFLP-Markem üblich ist; vgl. (14). Mit dieser Information kann dann bei den betroffenen Familien eine genetische Beratung bzw. eine pränatale Diagnose durchgeführt werden und zwar in analoger Weise, wie es für RFLP-Marker üblich ist. Der Einsatz des erfindungsgemäßen Verfahrens zu diesem Zweck ist vor allem deswegen sinnvoll, weil es sich auf DNA-Bereiche stützt, die mit einer vorhersagbaren Wahrscheinlichkeit polymorph sind, während die RFLP-Analyse auf zufällig gefundene Variationen angewiesen ist, die oft weit von dem Locus selbst entfernt liegen, was die Diagnosesicherheit herabsetzt.

Femer ist das erfindungsgemäße Verfahren zur Bestimmung von Polymorphismen in simplen oder kryptisch simplen DNA-Sequenzen von Tieren und Pflanzen geeignet. Daher können in der Tierzucht, z.B. bei Pferden, Hunden oder Rindern, die Verwandtschaftsverhältnisse zu hochwertigen Zuchtindividuen zuverlässig nachgewiesen werden.

Zusammenfassend liegt also der Vorteil des erfindungsgemäßen Verfahrens gegenüber den bisher bekannten Verfahren in seiner breiten Anwendbarkeit, schnellen Durchführbarkeit und in seiner hohen Empfindlichkeit. Der im erfindungsgemäßen Verfahren durchgeführte Amplifikationsschritt für die längenpolymorphen simplen oder kryptisch simplen DNA-Sequenzen macht einen unabhängigen Nachweisschritt, wie eine nachfolgende Hybridisierungsreaktion, überflüssig. Dadurch eignet sich das erfindungsgemäße Verfahren besonders gut zur Automatisierung und für Routinetests und Reihenuntersuchungen.

Die Figuren zeigen:

### Figur 1: Hybridisierung einer Genbank mit einer simplen DNA-Sequenz als Sondenmolekül.

Auf einer 12 x 12 cm großen Platte wurden ca. 20 000 vereinzelte Phagenclone ausplattiert und mit einem Sondenmolekül hybridisiert, das die simple Trinucleotidsequenz CAG/CTG enthält. Dabei werden etwa 300 bis 400 positive Signale erhalten. Die positiven Signale sind als Schwärzung erkennbar.

### Figur 2: Sequenz des in Beispiel 2 auf Polymorphismus getesteten Bereichs.

Die Bereiche, zu denen komplementäre Oligonucleotide synthetisiert wurden, sind mit einer Wellenlinie unterstrichen. Der Bereich der simplen DNA-Sequenz ist mit einer Doppellinie unterstrichen. Die direkte Wiederholung von 8 Nucleotiden ist mit zwei Pfeilen gekennzeichnet. Die Haelll-Spaltstelle ist kursiv markiert.

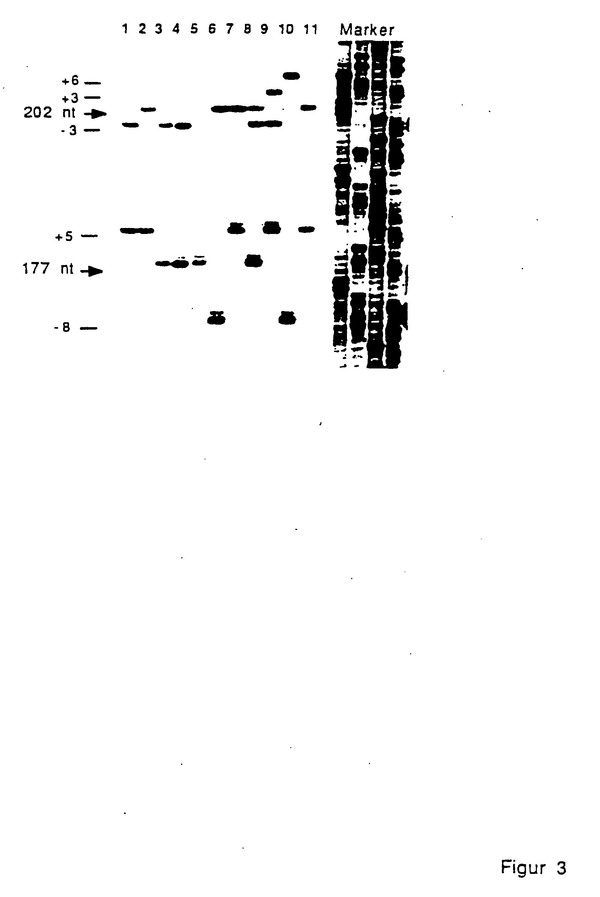

### Figur 3: Analyse der Längenvariationen in 11 Wildtypstämmen von Drosophila

Die mittels PCR amplifizierten und mit HaeIII gespaltenen DNA-Sequenzen sind in den Bahnen 1 bis 11 aufgetragen. Rechts ist eine Sequenzierungsreaktion aufgetragen, die als Längenmarker dient. Die Position der erwarteten Fragmente ist links mit Pfeilen markiert. Die Positionen der zusätzlich beobachteten Fragmentklassen ist mit Strichen markiert.

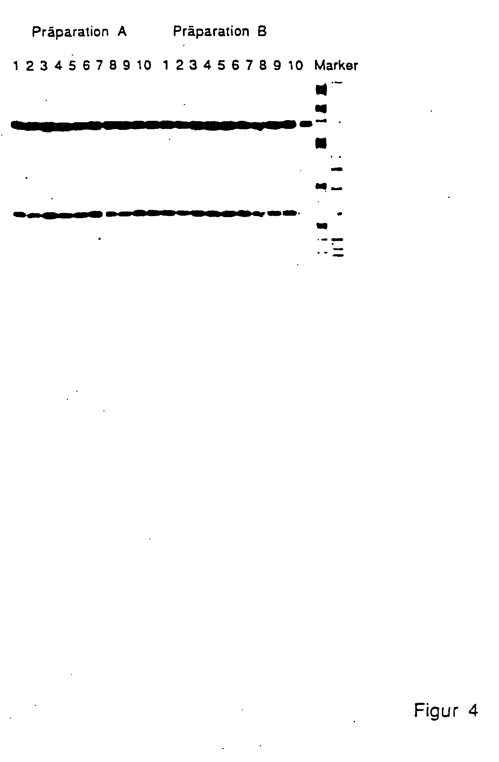

### Figur 4: Test auf Reproduzierbarkeit

Zehn unabhängige PCR-Ansätze mit der DNA-Präparation "A" des Drosophila-Stammes Nummer 3 wurden links aufgetragen, zehn unabhängige PCR-Ansätze mit der DNA-Präparation "B" des Drosophila-Stammes Nr. 3 wurden rechts aufgetragen. Ganz rechts sind Markerfragmente aus einer Sequenzierungsreaktion aufgetragen. Alle beobachteten Testbanden sind identisch.

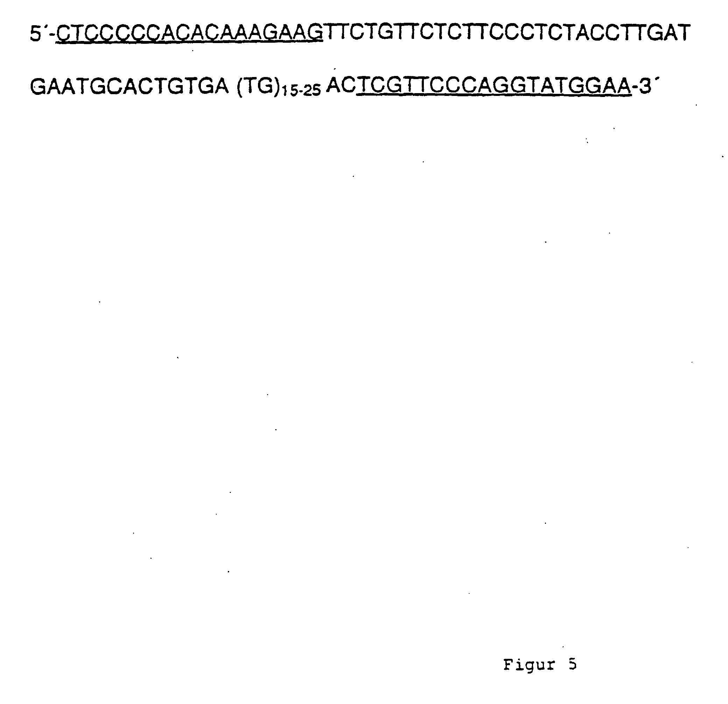

### Figur 5: Sequenz des in Beispiel 4 verwendeten DNA-Bereichs.

Die Bereiche, zu denen komplementäre Oligonucleotide synthetisiert wurden, sind mit einer Wellenlinie unterstrichen.

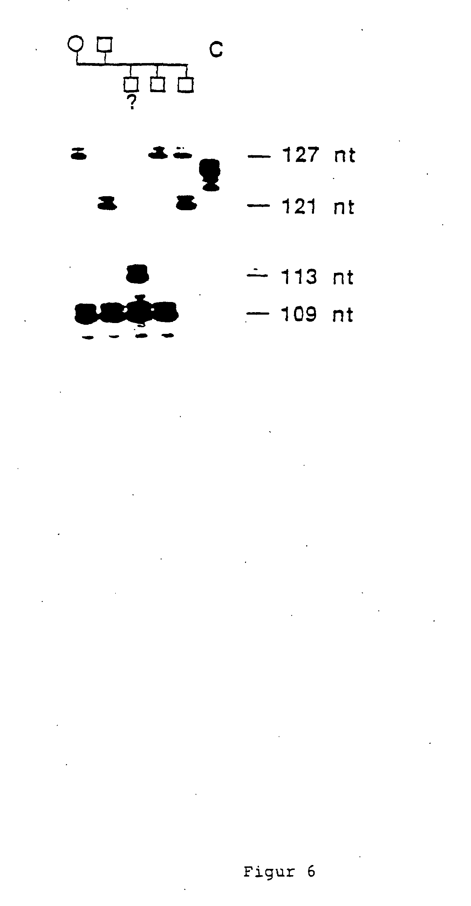

### Figur 6: Vaterschaftsanalyse beim Menschen.

Die mittels PCR amplifizierten und auf dem Gel aufgetrennten DNA-Fragmente sind gezeigt. In der ersten Spur ist die DNA der Mutter, in den folgenden Spuren die DNA des zu testenden Vaters, sowie der drei getesteten Kinder aufgetragen. In der sechsten Spur (markiert mit "C") ist eine Kontroll-DNA aufgetragen, die nur Größenklassen angeben soll. Die Hauptbanden und ihre Größenklassen sind am rechten Rand markiert.
Die Beispiele erläutern die Erfindung.

### Beispiel 1

### Isolierung von Clonen, die simple DNA-Sequenzen enthalten.

Drosophila-DNA wird mit der Restriktionsendonuclease EcoRI vollständig gespalten und die resultierenden Fragmente werden in den lambda-Vektor 641 cloniert. Eine nähere Beschreibung der angewendeten Methoden findet sich in (11). Somit wird eine Genbank erhalten, von der etwa 20 000 Phagen ausplattiert werden. Die entsprechenden vereinzelten Plaques werden auf einen Nitrocellulose-Filter übertragen und mit einem Sondenmolekül hybridisiert, das das simple DNA-Sequenzmotiv CAG/CTG enthält.

Die Filter werden bei 65°C hybridisert und gewaschen. Die Hybridisierungslösung enthält 5x SSPE, 5x Denhardt's Lösung, 0,1 % Natriumdodecylsulfat (SDS) und etwa 1 x 10⁶ cpm/ml radioaktiv (³²P) markierte DNA als Sondenmolekül. Die Waschlösung enthält 2 x SSPE und 0,1 % SDS (die Zusammensetzung von Denhardt's Lösung und SSPE ist in (11) beschrieben).

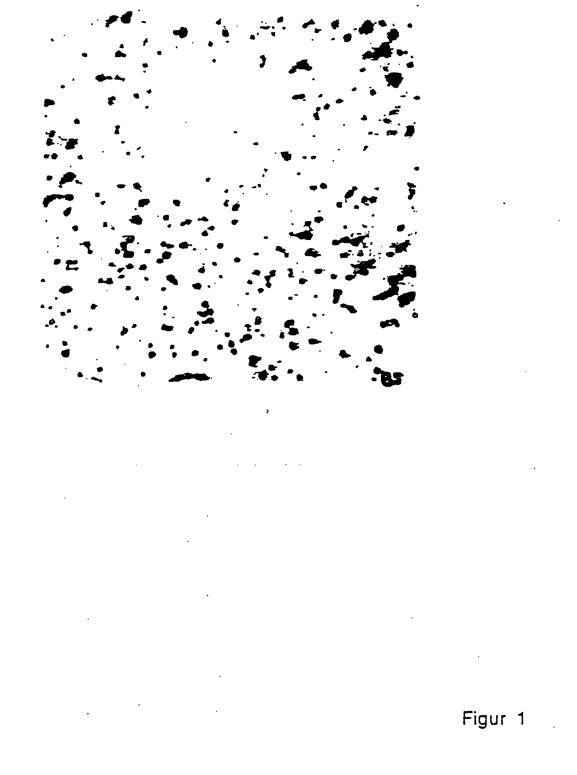

Etwa 300 bis 400 der ausgebildeten Plaques zeigen ein positives Signal; vgl. Figur 1. Von diesen Plaques werden einige gereinigt, es wird DNA isoliert und sequenziert. In den erhaltenen DNA-Sequenzen lassen sich Bereiche identifizieren, die die simple DNA-Sequenz CAG/CTG enthalten; vgl. (7).

### Beispiel 2

### Nachweis von Längenpolymorphismen

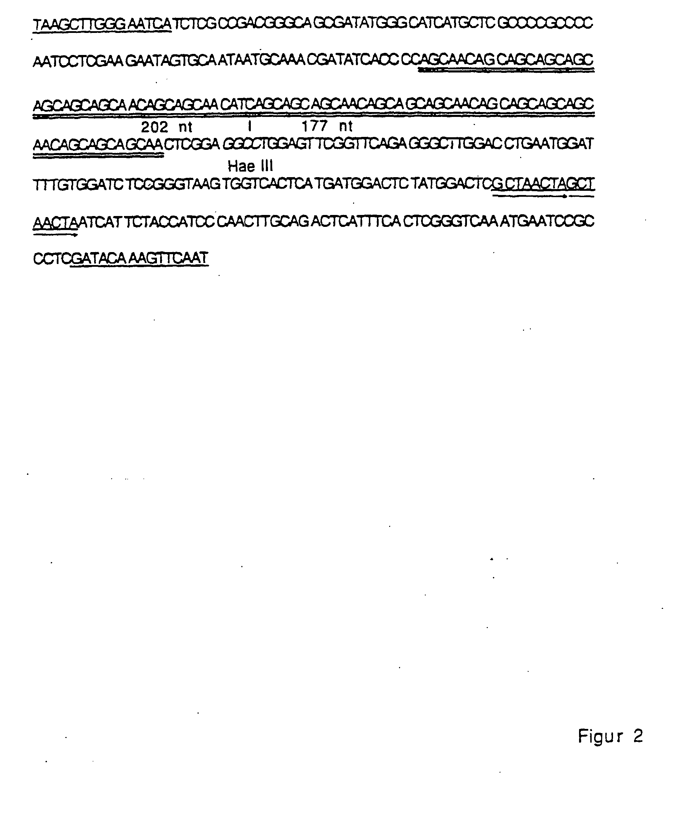

Für diesen Versuch wurde die in Figur 2 wiedergegebene und in (13) veröffentlichte DNA-Sequenz ausgewählt. Es wurden zwei Oligonucleotide mit den folgenden Sequenzen synthetisiert:

Oligonucleotid 1:  5'-TAAGCTTGGGAATCA-3'
Oligonucleotid 2:  5'-ATTGAACTTTGTATC-3'

Diese DNA-Sequenzen befinden sich unmittelbar am Beginn bzw. am Ende der in Figur 2 dargestellten Sequenz. Zur Verwendung als Primer werden die synthetisierten Oligonucleotide an ihrem 5'-Ende mit ³²P markiert. Sodann wird eine PCR-Reaktion mit den markierten Primern durchgeführt. Insgesamt werden 20 Zyklen durchgeführt, wobei jeweils 90 Sekunden bei 95°C denaturiert wird, 90 Sekunden bei 45°C angelagert wird und 120 Sekunden bei 72°C synthetisiert wird. Als zu untersuchende DNA's werden die genomischen DNA's von 11 Wildtypstämmen von Drosophila melanogaster aus verschiedenen Gebieten der ganzen Welt eingesetzt. Diese Drosophila-Wildtypstämme stammen ursprünglich von einzelnen fertilisierten Weibchen ab und wurden während der letzten 10 Jahre gesammelt. Nach der PCR-Reaktion werden die amplifizierten Fragmente mit der Restriktionsendonuklease Hae III gespalten. Dabei sollten üblicherweise zwei Fragmente entstehen, die eine Länge von 202 Nucleotiden bzw. 177 Nucleotiden aufweisen. Dieser Schritt ist für Routineexperimente normalerweise nicht erforderlich. Er dient hier lediglich der Verfeinerung der Analyse. Die entstehenden Fragmente werden auf einem 5prozentigen Sequenzierungsgel aufgetrennt, sodann wird das Gel getrocknet und ein Röntgenfilm wird mit dem getrockneten Gel belichtet. Die beiden erwarteten DNA-Fragmente zeigen einen ausgeprägten Polymorphismus in den verschiedenen Drosophila-Wildtypstämmen. Das die simple DNA-Sequenz enthaltende Fragment mit 202 Nucleotiden zeigt vier verschiedene Größenklassen; vgl. Figur 3. Diese Größenklassen sind jeweils um drei Nucleotide verschoben. Dies ist ausgehend von Rasterverschiebungen innerhalb der Wiederholung der Trinucleotide zu erwarten. In drei Fällen tauchen gleichzeitig zwei verschiedene Banden auf; vgl. Figur 3, Bahnen 5, 8 und 9. Dies ist dadurch erklärbar, daß in diploiden Organismen jeder Locus zweimal vorkommt und mit unterschiedlichen Allelen besetzt sein kann (sogenannter balancierter Polymorphismus). Die Bande des Fragments mit 177 Nucleotiden zeigt drei verschiedene Größenklassen, die 5 bzw. 8 Nucleotide auseinanderliegen; vgl. Figur 3. Die um 8 Nucleotide kürzere Bande ist vermutlich durch eine Deletion der in der DNA-Sequenz markierten Wiederholung von 8 Nucleotiden entstanden. Die Herkunft der längeren Bande ist unklar. Diese Deletionen bzw. Insertionen entsprechen denen, die man im Bereich einer kryptisch simplen DNA-Sequenz erwarten kann.

Die Mehrzahl der in diesem einfachen Experiment untersuchten Stämme ist bereits voneinander unterscheidbar. Nicht unterscheidbar sind lediglich die Stämme 2, 7 und 11 sowie 3 und 4. Für einen tatsächlichen Test würde man daher weitere Primer-Paare einsetzen. Beispielsweise könnten 20 bis 50 unabhängige DNA-Bereiche untersucht werden, um eine eindeutige Identifizierunq zu ermöglichen. Da die Größenklassen der Fragmente bei den einzelnen Drosophila-Wildtypstämme in sich einheitlich sind, ist davon auszugehen, daß die beobachteten Polymorphismen nicht mit so hoher Häufigkeit auftauchen, daß Verwandtschaftsbeziehungen nicht mehr feststellbar wären. Die Drosophila-Wildtypstämme stammen ja alle von jeweils einem einzelnen Ausgangspaar ab und für den Test wurde die DNA von mehreren 100 Individuen vereinigt. Wenn innerhalb dieser "Familien" eine Änderung des Musters aufgetreten wäre, müßte man mehr als maximal zwei Banden erwarten. Dies ist aber hier nicht der Fall. Daraus folgt, daß die beobachteten Längenklassen zumindest für einige Dutzend Generationen stabil sind.

### Beispiel 3

### Test auf Reproduzierbarkeit

Die beobachteten Längenvariationen könnten auch durch während des Versuchs auftretende Polymerasefehler verursacht werden. Um diese Möglichkeit auszuschließen und um gleichzeitig die allgemeine Reproduzierbarkeit nachzuweisen, wird der in Beispiel 2 durchgeführte Versuch mit zwei verschiedenen DNA-Präparationen des Drosophila-Stammes Nr. 3 in jeweils 10 unabhängigen Ansätzen wiederholt. Aus Figur 4 ist ersichtlich, daß alle Ansätze zu den gleichen Banden führen. Ähnliche Versuche wurden auch für andere Loci durchgeführt. Jedoch wurde in keinem Fall eine Veränderung der Bandenlänge beobachtet. Dies zeigt, daß das Verfahren zuverlässig reproduzierbar ist.

### Vergleichsbeispiel 1

### Vaterschaftstest beim Menschen

Es wird ein Primer-Paar verwendet, das eine Sequenzregion' aus dem autosomalen menschlichen Herzmuskelaktin-Gen flankiert. Diese Sequenz enthält eine simple Sequenz mit einer GT/CA Dinucleotidwiederholungs-Struktur (Figur 5). Als Primer werden folgende Oligonucleotide verwendet

Primer 1: 5'-CTCCCCCACACAAAGAAG-3'
Primer 2: 5'-TTCCATACCTGGGAACGA-3'

Primer 2 wird an seinem 5'-Ende mit ³²P markiert und beide Oligonucleotide werden dann für eine PCR-Reaktion verwendet. Es werden insgesamt 25 Zyklen mit einer Denaturierungsphase von 1 min bei 94°C, einer Anlagerungsphase von 2 min bei 45°C und einer Synthesephase von 1 min bei 72°C (letzte Synthesephase für 5 min) durchgeführt. Die Reaktionsprodukte werden dann auf einem 6%-igen denaturierenden Acrylamidgel aufgetrennt, das Gel wird getrocknet und exponiert. Das Ergebnis zeigt Fig. 6. Jedes der getesteten Individuen zeigt zwei Hauptbanden (zur Erklärung der weiteren Banden s.u.), ist also heterozygot für unterschiedliche Längenvarianten dieses Locus. Mutter und Vater haben die Längenvariante "109 nt" gemeinsam, unterscheiden sich aber in der anderen Variante, wobei die Mutter eine "127 nt" und der Vater eine "121 nt" Variante besitzt. Die Kinder müssen nun jeweils eine dieser Varianten von Vater und Mutter geerbt haben. Für zwei der Kinder ist dies auch der Fall, während das dritte Kind (markiert mit "?") eine neue "113 nt" Variante zeigt, die weder von der Mutter, noch vom getesteten Vater stammen kann. Man muß also davon ausgehen, daß dieses Kind einen anderen Vater hatte.

In Spur "C" ist eine clonierte Kontroll-DNA mitbehandelt worden, die nur eine Längenvariante besitzt. Wie die anderen Proben zeigt sie eine Hauptbande und mehrere Nebenbanden. Diese sind durch PCR-Artefakte bedingt, die während der Amplifikation entstehen. Es gibt dabei zwei Typen von Artefakten. Der erste Typ ist darauf zurückzuführen, daß die Taq-Polymerase die Tendenz besitzt, an den fertig synthetisierten DNA-Strang ein weiteres Nucleotid anzuhängen.

Dadurch entsteht die Bande, die ein Nucleotid oberhalb der Hauptbande läuft. Dieser Effekt ist von Reaktion zu Reaktion unterschiedlich stark ausgeprägt, aber für die Analyse des Bandenmusters nicht störend. Ein zweiter Typ von Artefakten entsteht durch "slippage" während des Amplifikationsprozesses. Dadurch entstehen die Banden, die im Dinucleotidabstand unterhalb der Hauptbanden zu sehen sind. Diese Artefaktbanden können sich störend auf die Analyse auswirken, wenn sie tatsächliche Längenvarianten überlagern.
Simple Sequenzen mit Trinucleotidwiederholungsmotiven zeigen diese Artefaktbanden nicht (vgl. Beispiel 2), da bei diesen Sequenzen "slippage" während der Amplifikation seltener ist.

### Literatur:

1: EP-A2 0 200 362
   1.a EP-A1 0 237 362
2. R.K. Saiki et al., Science 239 (1988), 487-491
3. A.J. Jeffreys et al., Nature 314 (1985), 67-73
4. A.J. Jeffreys et aL, Nature 316 (1985), 76-79
4.a Gill et al., Nature 318 (1985), 577-579
4.b Nakamura et al., Science 235 (1987), 1616-1622
5. EP 87 11 6408.3
5.a Higuchi et al., Nature 332 (1988), 543-546
6. D. Tautz, Doktorarbeit Universität Tübingen (1983)
7. D. Tautz und M. Renz, J. Mol. Biol. 172 (1984), 229-235
8. D. Tautz und M. Renz, Nucleic Acids Research 12 (1984), 4127 - 4138
9. D. Tautz et al., Nature 322 (1986), 652-656
10. G. Levinson und G.A. Gutman, Mol. Biol. and Evolution 4 (1987), 203 - 221
11. T. Maniatis, E.F. Fritsch und J. Sambrook, "Molecular Cloning, a Laboratory Manual", Cold Spring Harbor Laboratory, New York, 1982
12. LM. Smith et al., Nature 321 (1986), 674-679
13. K.A. Wharton et al., Cell 40 (1985), 55-62
14. Y.W. Kan und A.M. Dozy, Proc. Natl. Acad. Sci. USA 75 (1978), 5631-5635
15. Marx, Science 240 (1988), 1408 - 1410
16. Rollo et al., Chem. Abstr. 108(1) (1988), 154, Zusammenfassung Nr. 1552s

## Patentansprüche

1. Verfahren zur Analyse von Längenpolymorphismen in simplen oder kryptisch simplen DNA-Bereichen, bei dem man folgende Schritte durchführt:
(a) Anlagerung von mindestens einem Primer-Paar an mindestens einen spezifisch zu amplifizierenden Bereich der zu analysierenden DNA, wobei jeweils eines der Moleküle des Primer-Paares im wesentlichen komplementär ist zu einem der komplementären Stränge der 5'- bzw. 3'-Flanke einer simplen oder kryptisch simplen DNA-Sequenz, die Wiederholungseinheiten von mindestens drei Nucleotiden und höchstens sechs Nucleotiden enthält, die dutzend- bis hundertmal tandemartig oder mit Unterbrechungen wiederholt sind, und die Anlagerung in solcher Orientierung erfolgt, daß die bei einer Primer-gesteuerten Polymerisationsreaktion mit jeweils einem der beiden Primer gewonnenen Syntheseprodukte nach einer Denaturierung als Matrize zur Anlagerung des jeweils anderen Primers dienen können;
(b) Primer-gesteuerte Polymerasekettenreaktion; und
(c) Auftrennung und Analyse der Polymerasekettenreaktionsprodukte.

2. Verfahren nach Anspruch 1, bei dem die simple oder kryptisch simple DNA-Sequenz das Sequenz-Motiv CAG/CTG enthält.

3. Verfahren nach Anspruch 1 oder 2, bei dem die Moleküle des Primer-Paares in einem Abstand von 50 bis 500 Nucleotiden voneinander an die zu untersuchende DNA angelagert werden.

4. Verfahren nach einem der Ansprüche 1 bis 3, bei dem 2 bis 50 Primer-Paare eingesetzt werden.

5. Verfahren nach einem der Ansprüche 1 bis 4, bei dem die Primer eine Länge von 15 bis 25 Nucleotiden aufweisen.

6. Verfahren nach einem der Ansprüche 1 bis 5, bei dem die Lage der Primer-Paare so ausgewählt wird, daß die spezifischen Polymerasekettenreaktionsprodukte der Primer-Paare auf einem geeigneten Gel in einzelne Banden auftrennbar sind.

7. Verfahren nach einem der Ansprüche 1 bis 6, bei dem der Nachweis der spezifischen Polymerasekettenreaktionsprodukte durch radioaktive Markierung oder durch nicht-radioaktive Markierung, wie Fluoreszenzfarbstoffe, erfolgt.

8. Verfahren nach einem der Ansprüche 1 bis 7, bei dem die zu untersuchenden simplen oder kryptisch simplen DNA-Sequenzen in der Nähe oder innerhalb eines genetisch definierten Locus sitzen, so daß der gefundene Polymorphismus als Marker für diesen Locus dienen kann.

9. Kit zur Durchführung des Verfahrens nach einem der Ansprüche 1 bis 8, enthaltend Primer, die simple oder kryptisch simple DNA-Sequenzen flankieren und die gegebenenfalls radioaktiv oder fluoreszenz-markiert sind.

10. Kit nach Anspruch 9, enthaltend
(a) ein oder mehrere Gefäße mit einer äquimolaren Mischung von 1 bis 50 Oligonucleotidprimer-Paaren;
(b) ein Gefäß mit einem Enzym zur Polymerisation;
(c) ein Gefäß mit den vier Desoxynucleosidtriphosphaten; und
(d) ein Gefäß mit einer geeigneten Pufferstammlösung; und gegebenenfalls
(e) ein Gefäß mit einer Kontroll-DNA, die zum Testen der Komponenten des Kits geeignet sind.

11. Kit nach Anspruch 9 oder 10, bei dem die Primer die simplen oder kryptisch simplen DNA-Sequenzen in einem Abstand von 50 bis 500 Nucleotiden voneinander flankieren.

## Claims

1. A method for analyzing length polymorphisms in simple or cryptically simple DNA regions **characterized in that** the following steps are carried out:
(a) annealing at least one primer pair to at least one region to be specifically amplified of the DNA to be analyzed wherein one of the molecules of the primer pair is substantially complementary to one of the complementary strands of the 5' or 3' flank of a simple or cryptically simple DNA sequence which contains repeats of at least three nucleotides and not more than six nucleotides which are repeated a dozen to a hundred times in tandem repeats or with interruptions, and wherein the annealing occurs in such an orientation that the synthesis products obtained by a primer-directed polymerization reaction with one of said two primers can serve as a template for annealing the other primer after denaturation;
(b) primer-directed polymerase chain reaction; and
(c) separating and analyzing the polymerase chain reaction products.

2. The method according to claim 1 wherein the simple or cryptically simple DNA sequence contains the sequence motif CAG/CTG.

3. The method according to claim 1 or 2 wherein the molecules of the primer pair are annealed to the DNA to be analyzed in a distance of 50 to 500 nucleotides from each other.

4. The method according to any one of claims 1 to 3 wherein 2 to 50 primer pairs are used.

5. The method according to any one of claims 1 to 4 wherein the primers have a length of 15 to 25 nucleotides.

6. The method according to any one of claims 1 to 5 wherein the position of the primer pairs is selected such that the specific polymerase chain reaction products of the primer pairs are separable one from the other as individual bands on a suitable gel.

7. The method according to any one of claims 1 to 6 wherein the detection of the specific polymerase chain reaction products is carried out by radioactive labelling or by non-radioactive labelling such as fluorescent dyes.

8. The method according to any one of claims 1 to 7 wherein the simple or cryptically simple DNA sequences to be analyzed are located adjacent to or within a genetically defined locus such that the polymorphism found can serve as a marker for said locus.

9. A kit for performing the method according to any one of claims 1 to 8 containing primers flanking simple or cryptically simple DNA sequences and optionally being labelled with a radioactive or fluorescent label.

10. The kit according to claim 9 containing:
(a) one or more vessels containing an equimolar mixture of 1 to 50 oligonucleotide primer pairs;
(b) a vessel containing an enzyme for polymerization;
(c) a vessel containing the four deoxynucleoside triphosphates; and
(d) a vessel containing a suitable buffer stock solution; and optionally
(e) a vessel containing a control DNA suitable for assaying the components of the kit.

11. The kit according to claim 9 or 10 wherein the primers flank the simple or cryptically simple DNA sequences in a distance of 50 to 500 nucleotides from each other.

## Revendications

1. Procédé pour l'analyse de polymorphismes longitudinaux dans des domaines d'ADN simples ou cryptiques simples, dans lequel on effectue les étapes suivantes :
(a) fixation d'au moins une paire d'amorces à au moins un domaine, devant subir une amplification spécifique, de l'ADN devant être analysé, auquel cas chacune des molécules de la paire d'amorces est pour l'essentiel complémentaire de l'un des brins complémentaires de l'extrémité 5' ou 3' d'une séquence d'ADN simple ou cryptique simple, qui contient des motifs répétitifs d'au moins un nucléotide et d'au plus six nucléotides, motifs qui sont répétés douze à cent fois, en tandem ou avec des interruptions, la fixation étant réalisée selon une orientation telle que les produits de synthèse, qui dans le cadre d'une réaction de polymérisation pilotée par l'amorce sont chacun obtenus avec l'une des deux amorces, puissent servir après une dénaturation, en tant que matrice pour fixation de l'autre amorce ;
(b) réaction d'amplification en chaîne par polymérase, pilotée par l'amorce ; et
(c) séparation et analyse des produits de l'amplification en chaîne par polymérase.

2. Procédé selon la revendication 1, dans lequel la séquence d'ADN simple ou cryptique simple contient le motif de séquence CAG/CTG.

3. Procédé selon la revendication 1 ou 2, dans lequel les molécules de la paire d'amorces sont fixées à l'ADN devant être étudié, à une distance de 50 à 500 nucléotides l'une de l'autre.

4. Procédé selon l'une des revendications 1 à 3, dans lequel on utilise de 2 à 50 paires d'amorces.

5. Procédé selon l'une des revendications 1 à 4, dans lequel les amorces ont une longueur de 15 à 25 nucléotides.

6. Procédé selon l'une des revendications 1 à 5, dans lequel la position des paires d'amorces est choisie de façon que les produits spécifiques de la réaction d'amplification en chaîne par polymérase des paires d'amorces puissent être séparés en bandes individuelles sur un gel approprié.

7. Procédé selon l'une des revendications 1 à 6, dans lequel la détection des produits spécifiques de la réaction d'amplification en chaîne par polymérase s'effectue par radiomarquage, ou par un marquage non radioactif, tel que des colorants fluorescents.

8. Procédé selon l'une des revendications 1 à 7, dans lequel les séquences d'ADN simples ou cryptiques simples devant être étudiées se trouvent au voisinage ou à l'intérieur d'un site génétiquement défini, de sorte que le polymorphisme trouvé puisse jouer le rôle d'un marqueur pour ce site.

9. Coffret destiné à la mise en oeuvre du procédé selon l'une des revendications 1 à 8, contenant des amorces disposées latéralement par rapport à des séquences d'ADN simples ou cryptiques simples, et qui éventuellement sont radiomarquées ou marquées par fluorescence.

10. Coffret selon la revendication 9, contenant :
(a) un ou plusieurs récipients contenant un mélange équimolaire de 1 à 50 paires d'amorces oligonucléotidiques ;
(b) un récipient contenant une enzyme pour la polymérisation ;
(c) un récipient contenant les quatre désoxynucléosidetriphosphates : et
(d) un récipient contenant une solution-mère tampon appropriée ; et éventuellement
(e) un récipient contenant un ADN témoin ; qui conviennent à l'essai des constituants du coffret.

11. Coffret selon la revendication 9 ou 10, dans lequel les amorces flanquent les séquences d'ADN simples ou cryptiques simples à une distance de 50 à 500 nucléotides l'une de l'autre.
